# EUROPÄISCHE PATENTSCHRIFT

(11) **EP 0 389 504 B1**
(45) Veröffentlichungstag und Bekanntmachung des Hinweises auf die Patenterteilung: **11.08.1993**
(21) Anmeldenummer: 88909310.0
(22) Anmeldetag: 21.10.1988
(51) Int. Cl.: A61B 17/02

(54) **WUNDHAKEN**
RETRACTOR
ECARTEUR

(30) Priorität: 24.10.1987 DE 3736066
(43) Veröffentlichungstag der Anmeldung: 03.10.1990
(73) Patentinhaber: AESCULAP AG, D-78501 Tuttlingen (DE)
(72) Erfinder: LUTZE, Theodor, D-7201 Balgheim (DE); WEISSHAUPT, Dieter, D-7717 Immendingen (DE)
(74) Vertreter: Hoeger, Stellrecht & Partner
(86) Internationale Anmeldenummer: EP8800945
(87) Internationale Veröffentlichungsnummer: WO8903660

(56) Entgegenhaltungen:
- EP-A- 0 101 781
- DE-A- 3 301 890
- US-A- 4 562 832

## Beschreibung

Die Erfindung betrifft einen Wundhaken mit einem Wundhakenblatt, einem Griff, einem Lichtleiter zum Ausleuchten der Eingriffsstelle des Wundhakens und mit einer Kupplung zum Anschließen eines Lichtleitkabels an den Lichtleiter, wobei der Lichtleiter einen abgebogenen, rohrförmigen Mantel umfaßt, welcher durch den Griff hindurch und längs eines Teiles des Wundhakenblattes verläuft und in dem ein Glasfaserbündel eingebettet ist.

Es sind Wundhaken bekannt, bei denen ein Kunststoffwundhakenblatt unmittelbar mit einem Lichtleiter verbunden ist, so daß Licht durch das Kunststoffblatt hindurch bis zu der Vorderkante geleitet wird, an der es austritt (EP-B1 101 781). Ein solches Instrument wird üblicherweise als Nichtresterilisierbares Kunststoff-Einmalprodukt verwendet. Bei anderen bekannten Instrumenten dieser Art kann zwar der Griff wieder verwendet werden, jedoch werden die Kunststoffblätter als Wegwerfteile konzipiert (DE-OS 33 01 890).

Bei anderen bekannten Wundhaken ist an die Wundhaken ein Griff unmittelbar angeformt, der von einem ein Faserbündel aufnehmenden Rohr durchsetzt wird, Dieses Rohr ist an dem metallischen Wundhaken festgelötet oder geschweißt (Firmenprospekt "Fiberoptics for Surgery" Applied Fiberoptics, Inc., 0219/81, Seiten 5 ff). Das auf das Blatt aufgesetzte Rohr kann eine Sichteinschränkung hervorrufen, außerdem entstehen durch die Lötverbindungen Ecken und Kanten, die ein hygienisch einwandfreies Reinigen erschweren.

Es ist Aufgabe der Erfindung, einen Wundhaken der eingangs beschriebenen Art derart zu verbessern, daß alle Teile des Instrumentes resterilisierbar ausgebildet sind und daß zum Zwecke der Sterilisation eine einfache Zerlegung des Instrumentes in seine Einzelteile möglich ist.

Diese Aufgabe wird bei einem Wundhaken der eingangs beschriebenen Art erfindungsgemäß dadurch gelöst, daß der Mantel Befestigungselemente zur lösbaren Halterung des Wundhakenblattes am Mantel aufweist und daß der Handgriff auf das hintere Ende des Mantels lose aufgesteckt und in dieser Position durch ein lösbares Halteorgan festgelegt ist. Der die Lichtfaserbündel aufnehmende Mantel bildet also ein Gerüst für den Wundhaken, an dem einerseits lösbar das Wundhakenblatt befestigt ist, andererseits durch einfaches Aufstecken ein Griffteil, welches auf dem Mantel lösbar festlegbar ist. Die Teile können in einfacher Weise auseinandergenommen werden, und dann lassen sich alle Einzelteile in einfacher Weise reinigen und resterilisieren, wobei an keinem Teil Rücksprünge und Kanten entstehen, die Reinigungs- und Sterilisationsprobleme aufwerfen.

Eine besonders sichere Positionierung des Wundhakenblattes ergibt sich, wenn der Mantel eine ebene Anlagefläche für das Wundhakenblatt aufweist.

Vorzugsweise ist der Mantel zumindest in dem Teil, in dem das Wundhakenblatt an ihm anliegt, im Querschnitt rechteckig. Es ist vorteilhaft, wenn die Glasfasern zumindest an den beiden Enden des Mantels untereinander und mit dem Mantel verklebt sind. Dadurch ergibt sich eine vollständig dichte Ausbildung des Mantels, so daß das Mantelbauteil beliebig oft sterilisiert werden kann, ohne daß Beschädigungen der empfindlichen Glasfasern zu befürchten sind.

Bei der bevorzugten Ausführungsform ist vorgesehen, daß der Mantel eine quer zu seiner Längsrichtung verlaufende Kerbe oder Nut zur Aufnahme des griffseitigen Endes des Wundhakenblattes aufweist und daß am vorderen Ende des Mantels ein Rastorgan angeordnet ist, welches mit einem komplementären Rastorgan am Wundhakenblatt eine lösbare Rastverbindung bildet. Es ist dadurch in einfachster Weise möglich, das Wundhakenblatt an dem Mantel zu befestigen und von ihm wieder abzunehmen, wobei es trotzdem bei der Betätigung fest am Mantel gehalten ist, so daß mit dem Instrument insgesamt auch große Kräfte übertragen werden können. Dadurch wird es möglich, bei Bedarf das Wundhakenblatt rasch auszuwechseln, so daß derselbe Wundhaken nacheinander mit Wundhakenblättern der gewünschten Abmessungen verwendet werden kann.

Besonders vorteilhaft ist es, wenn das Rastorgan am Mantel ein keilförmiger Vorsprung ist, der an seiner Oberseite eine quer zur Ebene des Wundhakenblattes verlaufende Stützfläche aufweist, und wenn als komplementäres Rastorgan eine Ausnehmung im Wundhakenblatt angeordnet ist. Dabei kann vorzugsweise vorgesehen sein, daß im Wundhakenblatt auf der dem Mantel zugewandten Seite an die obere Kante der Ausnehmung eine abgeschrägte Vertiefung anschließt. Dadurch wird beim Einsetzen des Wundhakenblattes ein elastisches Einschnappen ermöglicht, wobei sich das Wundhakenblatt insgesamt in geringem Masse elastisch verformt. Nach dem Einschnappen sitzt das Wundhakenblatt form- und kraftschlüssig an den Mantel; zur Abnahme muß das freie Ende des Wundhakenblattes elastisch in Richtung auf den Griff gebogen werden, um die Rastverbindung wieder zu lösen.

Die Nut oder Kerbe zur Aufnahme des griffseitigen Endes des Wundhakenblattes kann in einer Verdickung des Mantels angeordnet sein, die vorzugsweise gleichzeitig einen vorderen Anschlag für den Griff bildet.

Weiterhin kann vorgesehen sein, daß das Wundhakenblatt an seinem griffseitigen Ende Führungsflächen aufweist, die bei eingesetztem Wundhakenblatt an beiden Seiten des Mantels anliegen. Dadurch wird einerseits das Einsetzen des Wundhakenblattes erleichtert, andererseits fixieren diese seitlichen Führungsflächen das Wundhakenblatt am Mantel gegen eine seitliche Verschiebung.

Bei dem weiteren bevorzugten Ausführungsbeispiel ist vorgesehen, daß auf das bei aufgestecktem Griff aus diesem hervorstehende Ende des Mantels zur Kupplung mit dem Lichtleitkabel ein Überwurfring aufschraubbar ist. Es handelt sich somit um ein Schnellverschluß zwischen dem Lichtleitkabel und dem die Lichtleitfasern aufnehmenden Mantel.

Dabei ist es besonders vorteilhaft, wenn der aufgeschraubte Überwurfring an dem Griff anliegt und diesen gegen einen vorderen Anschlag andrückt. Der die Schnellkupplung ausbildende Überwurfring dient dann gleichzeitig zur axialen Festlegung des Griffes auf dem Mantel, so daß umgekehrt nach dem Lösen der Kupplung ohne weiteres der Griff ausgetauscht werden kann.

Besonders vorteilhaft ist es, wenn das Wundhakenblatt im Anlagebereich am Mantel band- oder streifenförmig ausgebildet ist. Es legt sich dann über die gesamte Länge an den Mantel an und erfährt dadurch eine außerordentlich wirksame Stabilisierung. Andererseits ist durch diese Ausbildung ein elastisches Verbiegen in geringem Umfange möglich, so daß das Einsetzen und Abnehmen des Wundhakenblattes ermöglicht wird.

Die nachfolgende Beschreibung bevorzugter Ausführungsformen der Erfindung dient im Zusammenhang mit der Zeichnung der näheren Erläuterung.

Es zeigen:
- Fig. 1: Eine perspektivische Ansicht eines Wundhakens mit eingesetzten Wundhakenblatt (ausgezogene Linien) und mit abgenommenem Wundhakenblatt (strichpunktierte Linien);
- Fig. 2: Eine Schnittansicht längs Linie 2-2 in Fig. 1;
- Fig. 3: Eine Schnittansicht längs Linie 3-3 in Fig. 1;
- Fig. 4: Eine Draufsicht auf die Ausnehmung im Wundhakenblatt von der Mantelseite her gesehen und
- Fig. 5: der Endbereich eines abgewandelten Ausführungsbeispieles eines Wundhakenblattes mit seitlich vorstehenden Zinken.

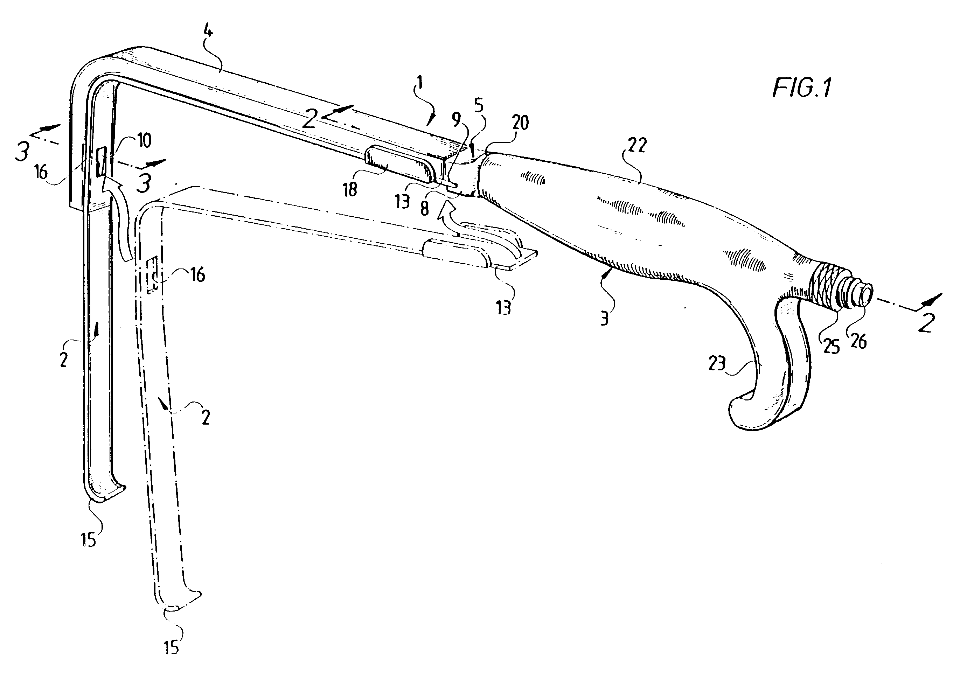

Der in Fig. 1 dargestellte Wundhaken umfaßt im wesentlichen drei Teile, nämlich ein rohrförmiges, metallisches Gehäuse mit rechteckigem Querschnitt, das an einem Ende rechtwinklig abgebogen ist und das im folgenden als Mantel 1 bezeichnet wird, des weiteren ein lösbar mit dem Mantel 1 verbundenes im wesentlichen L-förmiges, aus band- oder streifenförmigem Metall bestehendes Wundhakenblatt 2 sowie einen hohlen, auf den Mantel 1 aufsteckbaren Griff 3.

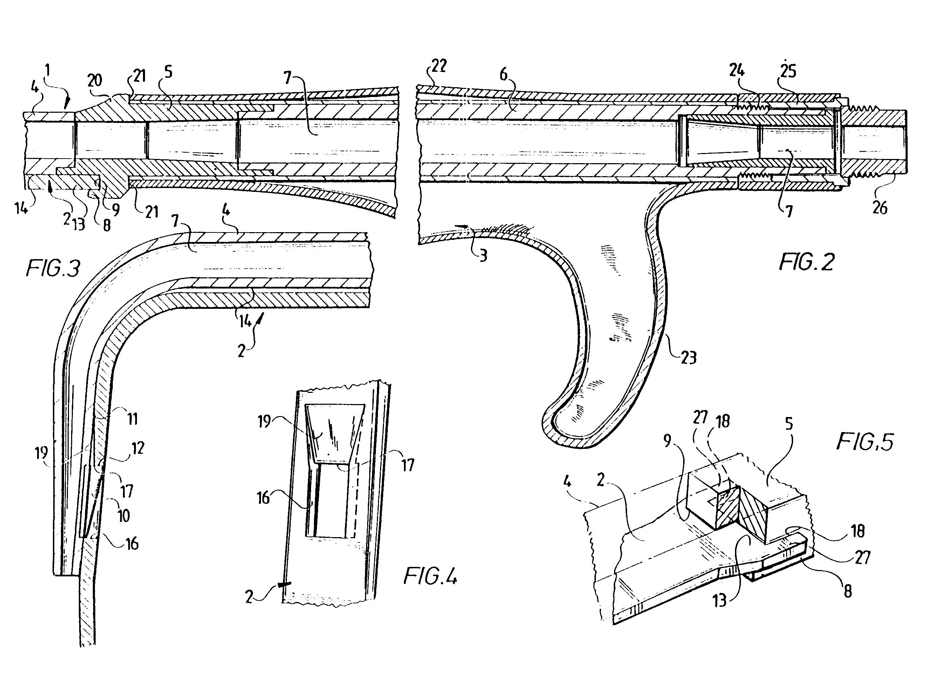

Der Mantel selbst ist bei dem dargestellten Ausführungsbeispiel aus drei Abschnitten zusammengesetzt, nämlich einem wundhakenseitigen vorderen Abschnitt 4, einem Mittelabschnitt 5 und einem normalerweise im Inneren des Griffes 3 angeordneten Griffabschnitt 6. Die drei Abschnitte des Mantels weisen einen im Querschnitt rechteckförmigen, bündig durchgehenden Kanal 7 auf, der sich an seinem wundhakenblattseitigen Ende im Querschnitt verjüngen kann (Fig. 3). Im Inneren dieses Kanals sind - in der Zeichnung nicht dargestellt - eine Vielzahl von Glasfasern eingebettet, die zumindest an den beiden Enden des Mantels 1 untereinander und mit der Innenwand des Mantels 1 kompakt verklebt sind, so daß der Kanal 7 nach außen vollständig abgedichtet ist und die Fasern in dem Mantel festgelegt und gegen jede Beschädigung gesichert sind.

Der Mittelabschnitt 5 weist an seinem unteren Ende einen Haltevorsprung 8 auf, der eine quer zur Längsrichtung des Mantels 1 verlaufende, sich unmittelbar an die ebene Unterseite des Mantels 1 anschließende Kerbe oder Nut 9 ausbildet, die zum vorderen Ende des Wundhakens hin offen ist. In der Nähe des vorderen Endes ist auf der dem Griff 3 zugewandten Seite des Mantels 1 ein keilförmiger Rastvorsprung 10 angeordnet, der an seiner Oberseite in einer senkrecht zur Mantelfläche 11 verlaufenden Stützfläche 12 endet.

Die Wundhakenblätter 2 können mit ihrem griffseitigen Ende 13 in die Nut 9 eingeschoben werden wobei die Wundhakenblätter 2 gegenüber der Unterseite 14 des Mantels 1 und gegenüber der Mantelfläche 11 zunächst einen Abstand aufweisen. Durch einen Druck auf das in Richtung des Griffes umgebogene freie Ende 15 des Wundhakenblattes 2 kann dieses in Richtung auf die Unterseite 14 und die Mantelfläche 11 verschwenkt werden, wobei die Nut 9 die Drehachse definiert. Dabei gleitet der eine Schenkel des Wundhakenblattes 2 an dem keilförmigen Rastvorsprung 10 entlang und wird dabei elastisch von der Mantelfläche 11 weggebogen, bis der Rastvorsprung 10 in eine rechteckige Rastausnehmung 16 im Wundhakenblatt 2 einschnappt. Die Rastausnehmung 16 ist dabei so angeordnet, daß die obere Kante 17 der Rastausnehmung 16 auf der Stützfläche 12 aufruht und dabei das Wundhakenblatt 2 flächig sowohl an die Unterseite 14 als auch an die Mantelfläche 11 des Mantels 11 anlegt. In dieser angelegten Position, die in Fig. 1 mit ausgezogenen Linien dargestellt ist, ist das Wundhakenblatt 2 am Mantel 1 festgelegt, so daß Kräfte vom Mantel 1 auf das Wundhakenblatt 2 übetragen werden können. Diese Festlegung wird durch seitliche Lappen oder Führungsflächen 18 erhöht, die sich am griffseitigen Ende des Wundhakenblattes 12 befinden und an den Seitenflächen des Mantels 1 anliegen.

Das Einschnappen des Wundhakenblattes 2 wird im übrigen dadurch erleichtert, daß sich an die obere Kante 17 der Rastausnehmung 16 auf der dem Mantel 1 zugewandten Seite eine abgeschrägte Vertiefung 19 anschließt (Fig. 3), so daß die Materialstärke des Wundhakenblattes 2 im Bereich der oberen Kante 17 geringer ist als an anderen Stellen. Dadurch kann das Wundhakenblatt 2 beim Anlegen leichter an dem Rastvorsprung 10 entlang gleiten.

Obwohl das Wundhakenblatt 2 am Mantel 1 so festgelegt ist, daß auch erhebliche Kräfte übertragen werden können, kann es in einfacher Weise wieder vom Mantel 1 gelöst werden, in dem das Wundhakenblatt 2 am freien Ende 15 in Richtung auf den Griff 3 verbogen wird. Dadurch wird es möglich, das Wundhakenblatt 2 um die durch die Nut 9 gebildete Drehachse entgegen dem Uhrzeigersinn (Fig. 1) an dem Rastvorsprung 10 vorbei zu verschwenken, so daß anschließend das Wundhakenblatt 2 aus der Nut 9 herausgezogen werden kann. Auf diese Weise ist ein Wechsel des Wundhakenblattes 2 jederzeit möglich.

Der Haltevorsprung 8 und eine Verdickung 20 auf der Oberseite des Mittelabschnittes 5 bilden gemeinsam eine axiale Anschlagfläche 21 für den von der Rückseite auf den Mantel 1 aufgeschobenen Griff 3 aus. Dieser Griff 3 selbst hat ein im wesentlichen rohrförmiges Gehäuse 22, an dessen Unterseite ein anatomisch ausgeformter Haken 23 angeformt ist. Der Griff 3 ist insgesamt etwas kürzer als der Griffabschnitt 6 des Mantels 1, so daß der Mantel 1 auf der Rückseite aus dem Griff 3 her vorsteht. In diesem Bereich trägt der Mantel 1 ein Außengewinde 26, auf welches eine Überwurfmutter 25 eines Lichtleitkabels 26 so aufgeschraubt werden kann, daß die Stirnseiten des Lichtleitkabels 26 und des Mantels 1 dicht aneinander liegen (Fig. 2). Außerdem legt sich dabei die Überwurfmutter 25 an den Griff 3 an und drückt diesen in axialer Richtung gegen die Anschlagfläche 21, so daß durch die Überwurfmutter 25 der Griff 3 auf dem Mantel 1 in axialer Richtung festgelegt wird. Gegen eine Drehung ist der Griff 3 aufgrund des rechteckigen Querschnittes des Mantels 1 gesichert.

Der die Glasfasern aufnehmende Mantel 1 bildet somit das Hauptteil des gesamten Instrumentes, an dem lösbar Wundhakenblatt, Griff und Lichtleitkabel festgelegt werden können. Diese Teile lassen sich insbesondere zum Sterilisieren, aber auch während des Operationsbetriebes zum Auswechseln des Wundhakenblattes leicht abnehmen und austauschen. Das Lichtaustrittsende des Mantels 1 befindet sich bei dem in der Zeichnung dargestellten Ausführungsbeispiel im oberen Drittel des senkrecht abgebogenen Schenkels des Wundhakenblattes 2. Grundsätzlich könnte dieses Ende auch eine andere Position längs dieses Schenkels einnehmen.

Bei dem in Fig. 5 dargestellten Ausführungsbeispiel sind die Lappen oder Führungsflächen 18 ersetzt durch nach hinten über das Ende des Wundhakenblattes 2 vorstehende Zinken 27, die seitlich den Haltevorsprung 8 umgreifen und somit das Wundhakenblatt 2 gegen eine seitliche Verschiebung sichern. Die Innenflächen der Zinken 27 bilden dabei die Führungsflachen 18, diese Führungsflächen 18 können zum freien Ende der Zinken 27 hin leicht divergieren, so daß beim Einsetzen des Wundhakenblattes eine zentrierende Wirkung eintritt.

## Patentansprüche

1. Wundhaken mit einem Wundhakenblatt (2), einem Handgriff (3), einem Lichtleiter zum Ausleuchten der Eingriffsstelle des Wundhakens und min einer Kupplung zum Anschließen eines Lichtleitkabels (26) an den Lichtleiter, wobei der Lichtleiter einen abgebogenen, rohrförmigen Mantel (1) umfaßt, welcher durch den Handgriff hindurch und längs eines Teiles des Wundhakenblattes verläuft und in dem ein Glasfaserbündel eingebettet ist,
**dadurch gekennzeichnet**,
daß der Mantel (1) Befestigungselemente (9, 10) zur lösbaren Halterung des Wundhakenblattes (2) am Mantel (1) aufweist und daß der Handgriff (3) auf das hintere Ende (6) des Mantels (1) lose aufgesteckt und in dieser Position durch ein lösbares Halteorgan (25) festgelegt ist.

2. Wundhaken nach Anspruch 1,
**dadurch gekennzeichnet**,
daß der Mantel (1) eine ebene Anlagefläche (11, 14) für das Wundhakenblatt (2) aufweist.

3. Wundhaken nach Anspruch 1 oder 2,
**dadurch gekennzeichnet**,
daß der Mantel (1) zumindest in dem Teil (4), an dem das Wundhakenblatt (2) anliegt, im Querschnitt rechteckig ist.

4. Wundhaken nach einem der voranstehenden Ansprüche,
**dadurch gekennzeichnet**,
daß die Glasfasern zumindest an den beiden Enden des Mantels (1) untereinander und mit dem Mantel (1) verklebt sind.

5. Wundhaken nach einem der voranstehenden Ansprüche,
**dadurch gekennzeichnet**,
daß der Mantel (1) eine quer zu seiner Längsrichtung verlaufende Kerbe oder Nut (9) zur Aufnahme des griffseitigen Endes (13) des Wundhakenblattes (2) aufweist und daß am vorderen Ende des Mantels (1) ein Rastorgan (10) angeordnet ist, welches mit einem komplementären Rastorgan (16) am Wundhakenblatt (2) eine lösbare Rastverbindung bildet.

6. Wundhaken nach Anspruch 5,
**dadurch gekennzeichnet**,
daß das Rastorgan (10) am Mantel (1) ein keilförmiger Vorsprung ist, der an seiner Oberseite eine quer zur Ebene des Wundhakenblattes (2) verlaufende Stützfläche (12) aufweist, und daß als komplementäres Rastorgan (16) eine Ausnehmung im Wundhakenblatt (2) angeordnet ist.

7. Wundhaken nach Anspruch 6,
**dadurch gekennzeichnet**,
daß sich im Wundhakenblatt (2) auf der dem Mantel (1) zugewandten Seite an die obere Kante (17) der Ausnehmung (16) eine abgeschrägte Vertiefung (19) anschließt.

8. Wundhaken nach einem der Ansprüche 5 bis 7,
**dadurch gekennzeichnet**,
daß die Nut oder Kerbe (9) in einer Verdickung (5) des Mantels (1) angeordnet ist.

9. Wundhaken nach Anspruch 8,
**dadurch gekennzeichnet**,
daß die Verdickung (5) einen vorderen Anschlag (21) für den Griff (3) bildet.

10. Wundhaken nach einem der voranstehenden Ansprüche,
**dadurch gekennzeichnet**,
daß das Wundhakenblatt (2) an seinem griffseitigen Ende (13) Führungsflächen (18) aufweist, die bei eingesetztem Wundhakenblatt (2) an beiden Seiten des Mantels (1) anliegen.

11. Wundhaken nach einem der voranstehenden Ansprüche,
**dadurch gekennzeichnet**,
daß auf das bei aufgestecktem Griff (3) aus diesem hervorstehenden Ende des Mantels (1) zur Kupplung mit dem Lichtleitkabel (26) ein Überwurfring (25) aufschraubbar ist.

12. Wundhaken nach Anspruch 11,
**dadurch gekennzeichnet**,
daß der aufschraubbare Überwurfring (25) an dem Griff (3) anliegt und diesen gegen einen vorderen Anschlag (21) andrückt.

13. Wundhaken nach einem der voranstehenden Ansprüche,
**dadurch gekennzeichnet**,
daß das Wundhakenblatt (2) im Anlagebereich am Mantel (1) band- oder streifenförmig ausgebildet ist.

14. Wundhaken nach einem der Ansprüche 10 bis 13,
**dadurch gekennzeichnet**,
daß die Führungsflächen (18) durch die einander gegenüberliegenden Innenflächen von Zinken (27) gebildet werden, die am Ende des Wundhakenblattes (2) über dieses vorstehen und die Nut oder Kerbe (9) des Mantels (1) seitlich umgreifen.

## Claims

1. A wound retractor having a wound retractor blade (2), a handle (3), a light guide for illumination of the intervention site of the wound retractor and having a coupling for the connection of a optical cable (26) to the light guide, the latter comprising a bent, tubular casing (1) which runs through the handle and along a portion of the wound retractor blade and in which there is embedded a glass-fibre bundle, characterised in that the casing (1) has securing elements (9, 10) for detachable fastening of the wound retractor blade (2) to the casing (1), and in that the handle (3) is loosely placed onto the rear end (6) of the casing (1) and is made fast in this position by means of a detachable retaining member (25).

2. A wound retractor in accordance with Claim 1, characterised in that the casing (1) has a plane contact surface (11, 14) for the wound retractor blade (2).

3. A wound retractor in accordance with Claim 1 or 2, characterised in that the casing (1) is - at least in the portion (4) to which the wound retractor blade (2) is adjacent - rectangular in cross-section.

4. A wound retractor in accordance with any one of the preceding Claims, characterised in that the glass fibres are -at least at the two ends of the casing (1) - stuck to one another and to the casing (1).

5. A wound retractor in accordance with any one of the preceding Claims, characterised in that the casing (1) has a groove or channel (9) which runs at right angles to its longitudinal direction and is for receiving the end (13) of the wound retractor blade (2) which is nearest the handle, and in that on the front end of the casing (1) there is arranged a locking member (10) which with a complementary locking member (16) on the wound retractor blade (2) forms a detachable locking connection.

6. A wound retractor in accordance with Claim 5, characterised in that the locking member (10) on the casing (1) is a wedge-shaped projection having on its upper side a supporting surface (12) running transversely to the plane of the wound retractor blade (2), and in that a recess is arranged in the wound retractor blade (2) as a complementary locking member (16).

7. A wound retractor in accordance with Claim 6, characterised in that in the wound retractor blade (2), on the side facing the casing (1), an inclined cavity (19) is adjacent to the upper edge (17) of the recess (16).

8. A wound retractor in accordance with any one of Claims 5 to 7, characterised in that the channel or groove (9) is arranged in an enlargement (5) of the casing (1).

9. A wound retractor in accordance with Claim 8, characterised in that the enlargement (5) forms a front stop (21) for the handle (3).

10. A wound retractor in accordance with any one of the preceding Claims, characterised in that the wound retractor blade (2) has guide surfaces (18) at its end (13) nearest the handle, these guide surfaces being in contact with both sides of the casing (1) when the wound retractor blade (2) has been inserted.

11. A wound retractor in accordance with any one of the preceding Claims, characterised in that for coupling with the optical cable (26) a collet (25) can be screwed onto the casing (1) end which projects from the handle (3) when the latter is attached.

12. A wound retractor in accordance with Claim 11, characterised in that the screw-on collet (25) is in contact with the handle (3) and presses the latter against a front stop (21).

13. A wound retractor in accordance with any one of the preceding Claims, characterised in that the wound retractor blade (2) is ribbon-shaped or strip-shaped in the contact region with the casing (1).

14. A wound retractor in accordance with any one of Claims 10 to 13, characterised in that the guide surfaces (18) are formed by the facing inner surfaces of prongs (27) which at the end of the wound retractor blade (2) project above the latter and laterally grip around the channel or groove (9) of the casing (1).

## Revendications

1. Ecarteur comprenant une lame d'écarteur (2), une poignée (3), un conducteur de lumière servant à éclairer le point d'attaque de l'écarteur, et un raccord servant à raccorder un câble conducteur de lumière (26) au conducteur de lumière, le conducteur de lumière comprenant une gaine (1) de forme tubulaire, recourbée, qui traverse la poignée et s'étend le long d'une partie de la lame de l'écarteur, et dans laquelle est noyé un faisceau de fibres de verre,
caractérisé
en ce que la gaine (1) présente des éléments de fixation (9, 10) servant a' fixer la lame (2) de l'écarteur à la gaine (1) de façon démontable et en ce que la poignée (3) est emmanchée librement sur l'extrémité arrière (6) de la gaine (1) et est immobilisée dans cette position par un organe de retenue démontable (25).

2. Ecarteur selon la revendication 1,
caractérisé
en ce que la gaine (1) présente une surface d'appui plane (11, 14) pour la lame (2) de l'écarteur.

3. Ecarteur selon la revendication 1 ou 2,
caractérisé
en ce que la gaine (1) est de forme rectangulaire en section transversale, du moins dans la partie (4) contre laquelle s'applique la lame (2) de l'écarteur.

4. Ecarteur selon une des revendications précédentes,
caractérisé
en ce que les fibres de verre sont collées les unes aux autres et collées à la gaine (1), au moins aux deux extrémités de la gaine (1).

5. Ecarteur selon une des revendications précédentes,
caractérisé
en ce que la gaine (1) présente une rainure ou entaille (9) s'étendant transversalement à sa direction longitudinale, pour recevoir l'extrémité (13) côté poignée de la lame (2) de l'écarteur et en ce qu'à l'extrémité avant de la gaine (1), est disposé un organe de verrouillage (10) qui forme un assemblage de verrouillage démontable en combinaison avec un organe de verrouillage complémentaire (16) porté par la lame (2) de l'écarteur.

6. Ecarteur selon la revendication 5,
caractérisé
en ce que l'organe de verrouillage (10) porté par la gaine (1) est une saillie en forme de coin qui présente, sur son côté supérieur une surface d'appui (12) s'étendant transversalement au plan de la lame (2) de l'écarteur et en ce qu'une ouverture est pratiquée dans la lame (2) de l'écarteur pour servir d'organe de verrouillage complémentaire (16).

7. Ecarteur selon la revendication 6,
caractérisé
en ce que, dans la lame (2) de l'écarteur, se trouve une encoche oblique (19) formée sur la face dirigée vers la gaine (1), à la suite du bord supérieur (17) de l'ouverture (16).

8. Ecarteur selon une des revendications 5 à 7,
caractérisé
en ce que la rainure ou entaille (9) est prévue dans un renflement (5) de la gaine (1).

9. Ecarteur selon la revendication 8,
caractérisé
en ce que le renflement (5) forme une butée avant (21) pour la poignée (3).

10. Ecarteur selon une des revendications précédentes,
caractérisé
en ce que la lame (2) de l'écarteur présente, à son extrémité (13) côté poignée, des surfaces de guidage (18) qui s'appliquent contre les deux côtés de la gaine (1) lorsque la lame (2) de l'écarteur est en place.

11. Ecarteur selon une des revendications précédentes,
caractérisé
en ce qu'une bague d'accouplement (25) peut être vissée sur l'extrémité de la gaine (1) qui émerge de la poignée (3) lorsque cette poignée est emmanchée, pour le raccordement à un câble conducteur de lumière (26).

12. Ecarteur selon la revendication 11,
caractérisé
en ce que la bague d'accouplement (25) qui peut être vissée est appuyée contre la poignée (3) et applique cette dernière contre une butée avant (21).

13. Ecarteur selon une des revendications précédentes,
caractérisé
en ce que la lame (2) de l'écarteur est en forme de bande ou de lamelle dans la région d'appui contre la gaine (1).

14. Ecarteur selon une des revendications 10 à 13,
caractérisé
en ce que les surfaces de guidage (18) sont formées par les surfaces internes, en regard l'une de l'autre, de griffes (27) qui font saillie à l'extrémité de la lame (2) de l'écarteur au-delà de cette extrémité et qui encadrent latéralement la rainure ou entaille (9) de la gaine (1).
